(19) **Europäisches Patentamt European Patent Office Office européen des brevets**

(11) **EP 4 359 070 B1**

(12) **EUROPEAN PATENT SPECIFICATION**

(45) Date of publication and mention
of the grant of the patent:
**04.06.2025   Bulletin 2025/23**

(21) Application number: **22734265.6**

(22) Date of filing: **14.06.2022**

(51) International Patent Classification (IPC):
***A61N 5/10*** *(2006.01)*

(52) Cooperative Patent Classification (CPC):
**A61N 5/103; A61N 5/1047;** A61N 2005/1041

(86) International application number:
**PCT/EP2022/066190**

(87) International publication number:
**WO 2022/268576 (29.12.2022 Gazette 2022/52)**

(54) **MACHINE LEARNING APPROACH FOR SOLVING BEAM ANGLE OPTIMIZATION**

MASCHINENLERNANSATZ ZUR LÖSUNG DER STRAHLWINKELOPTIMIERUNG

APPROCHE D'APPRENTISSAGE AUTOMATIQUE POUR RÉSOUDRE UNE OPTIMISATION D'ANGLE DE FAISCEAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.06.2021   US 202117356235**

(43) Date of publication of application:
**01.05.2024   Bulletin 2024/18**

(73) Proprietor: **Siemens Healthineers International
AG
6312 Steinhausen (CH)**

(72) Inventors:
• **PRINC, Santiago Gaspar
5400 Baden (CH)**
• **NIEMELAE, Perttu
02180 Espoo (FI)**
• **JYSKE, Tuomas
01370 Vantaa (FI)**
• **VAN DER STRAETEN, Reynald
1180 Ukkel (BE)**

(74) Representative: **Foster, Mark Charles et al
Mathisen & Macara LLP
Charta House
30-38 Church Street
Staines-upon-Thames, Middlesex TW18 4EP
(GB)**

(56) References cited:
**WO-A1-2022/129144      US-A1- 2021 142 476
US-A1- 2022 184 419**

• **OGUNMOLU OLALEKAN ET AL: "Deep BOO!
Automating Beam Orientation Optimization in
Intensity-Modulated Radiation Therapy",
INTERNATIONAL WORKSHOP ON THE
ALGORITHMIC FOUNDATIONS OF ROBOTICS, 8
May 2020 (2020-05-08), XP055962824, DOI:
10.1007/978-3-030-44051-0_20**
• **AZAR SADEGHNEJAD-BARKOUSARAIE ET AL:
"A reinforcement learning application of guided
Monte Carlo Tree Search algorithm for beam
orientation selection in radiation therapy",
ARXIV.ORG, CORNELL UNIVERSITY LIBRARY,
201 OLIN LIBRARY CORNELL UNIVERSITY
ITHACA, NY 14853, 14 April 2020 (2020-04-14),
XP081644215**

## Description

### TECHNICAL FIELD

This application relates generally to using artificial intelligence modeling to optimize beam angles in radiation therapy treatment procedures.

### BACKGROUND

Radiotherapy (radiation-based therapy) is used as a cancer treatment by emitting high doses of radiation that can kill cells or shrink a tumor. Due to the extreme nature of the radiation emitted from the radiation therapy machine, and in particular, of proton therapy, it is imperative that treatment attributes are precisely calculated and followed. For example, a beam angle defines a direction in which a radiotherapy machine emits radiation particles. The beam angle should be positioned (or set) such that the target region of a patient's anatomy that is intended to receive radiation (e.g., a tumor, referred to as the planning target volume (PTV)) receives enough radiation to kill the cancerous cells during the radiotherapy treatment. However, other organs or anatomical regions that are adjacent to, or surrounding, the PTV can be in the way of radiation beams (directed via the beam angle) and can receive enough radiation to damage or harm such organs or anatomical regions. These organs or anatomical regions are referred to as organs at risk (OARs).

Usually a physician or a radiologist identifies both the PTV and the OARs prior to radiotherapy using, for example, computed tomography (CT) images, cone beam CT images (CBCT), four-dimensional CT images (e.g., CT images over time), magnetic resonance imaging (MRI) images, positron emission tomography (PET) images, ultrasound images, images obtained via some other imaging modality, or a combination thereof. The physician or the radiologist may manually mark the PTV and/or the OARs on the medical images of the patient.

Radiation therapy treatment attributes refer to attributes of how the patient's treatment is implemented. Radiation therapy treatment attributes include attributes of a radiation therapy machine while the patient is receiving the prescribed radiotherapy dose and other radiation parameters (e.g., radiation intensity, beam angle, radiation type, number of beam fields (field geometry)) indicating how the dosage is delivered to the patient's anatomy. Using the medical images of the patient as well as the identified PTV and the OARs, a team of medical professionals (e.g., physicians, radiologists, oncologists, radiology technicians, other medical personnel or a combination thereof) determines the beam angles and other radiation parameters and radiation therapy treatment attributes to be used during the radiotherapy treatment. In determining these and other parameters, the medical professional attempts to set the beam angles in a treatment plan to achieve a radiation dose distribution to deliver to the patient that meets predefined criteria (also referred to herein as the clinical goals). Such clinical goals can include dosimetric metrics (e.g., predefined radiation dose thresholds, ranges for the PTV and the OARs, sensitivities of the PTV and/or OARs), a robustness measure, relative biological effects, and linear energy transfer metrics, among others.

Conventionally, beam angle optimization and other radiation therapy treatment attributes and radiation parameters are identified as part of a treatment plan for particular patients with particular tumors. For various types of tumors, medical professionals have determined, based on past experiences and trial and error, common beam angles and other radiation parameters. However, this conventional method is inefficient because it is error-prone, and relies heavily on the medical professional's subjective understanding and skills. Furthermore, this conventional process is time-consuming and tedious.

Document "Deep BOO! Automating Beam Orientation Optimization in Intensity-Modulated Radiation Therapy." (Ogunmolu, O., Folkerts, M., Nguyen, D., Gans, N., Jiang, S. (2020). In: Morales, M., Tapia, L., Sánchez-Ante, G., Hutchinson, S. (eds) Algorithmic Foundations of Robotics XIII. WAFR 2018. Springer Proceedings in Advanced Robotics, vol 14. Springer, Cham.) discloses a method of beam angle optimization comprising:
executing, by at least one processor, a machine learning model that receives an input of data associated with a treatment plan for a patient and outputs a beam angle for the patient indicating a direction of radiation into the patient;

- wherein the machine learning model iteratively calculates a reward, using a policy, for a possible beam angle for the training treatment plan in the training dataset, and wherein the machine learning model iteratively increases a summation of rewards until the policy satisfies an accuracy threshold;
- transmitting, by the at least one processor, the beam angle to a second processor.

### SUMMARY

According to one aspect of the invention, there is provided a computer-implemented method of beam angle optimization according to claim 1.

According to a second aspect of the invention, there is provided a system according to claim 9. Optional features are defined in the dependent claims.

For the aforementioned reasons, there is a need to optimize the beam angles in a treatment plan to improve the resulting quality of the treatment plan. There is a need for a beam angle optimization system that optimizes beam angles and other radiation parameters given a treatment plan. The task of learning how to adapt beam angles to better suit specific patients is translated from being a medical professional task, into being a task executed using a trainable machine learning model. As discussed herein, the angle of radiation beams administered to a patient is optimized to improve the clinical outcome of the targeted radiation during treatment. Disclosed herein are systems and methods capable of recommending beam angle modifications to optimize the patient's treatment plan in a manner that does not overrule medical professionals. In some implementations, a medical professional may propose an initial treatment plan to be optimized by the systems and methods described herein. In other implementations, systems and methods disclosed herein may not depend on a medical professional's subjective skills and understanding. For example, a processor may utilize the systems and methods described herein to optimize an initial treatment plan (e.g., treatment templates provided for a patient). The beam angles are optimized using an end-to-end machine learning model such that the beam angles can be used for guiding clinical plan optimization and to save calculation time. Rather than optimizing certain aspects of the beam angle or generating possible beam angles using an algorithm, the end-to-end machine learning model holistically optimizes the beam angle by ingesting, in some implementations, a point cloud representation of the structures segmented from medical images, clinical goals, and a treatment plan. In other implementations, an automatic image segmentation module may automatically segment PTVs and/or OARs in medical images. The medical images may be segmented, or otherwise analyzed to identify different contrasts in the medical image. The segmented medical images may subsequently be ingested by the end-to-end machine learning model. In yet other implementations, the end-to-end system may ingest medical images. The machine learning model recommends modifications to a base treatment plan (including beam angles) at a higher response time (once trained), is scalable (e.g., may be implemented using federated learning to update a central policy model), and may be extended (e.g., the core policy model is modular and can be modified with little to no impact beyond retraining). A flexible framework may be applied to many manifestations of the beam angle optimization problem. For example, beam angles may be optimized with respect to proton radiation as well as photon radiation.

The machine learning model replicates (or simulates) the way in which a dosimetrist would gain expertise on how to position the beams, and subsequently uses the trained model to recommend beam angle modifications that are predicted to result in a more performant treatment plan. The machine learning model receives a treatment plan that may include initial beam angles, PTV information, OAR information, medical images and/or various clinical goals. An environment, such as the anatomical structure of the patient and the positioning of the radiation fields, may be external to the machine learning model. Representations of the environment may be fed into the machine learning model. Agents operating in parallel on each environment, asynchronously select actions for the corresponding environment based on global policies and values. The machine learning model outputs a tensor indicating revised (or optimized, improved, and/or recommended) modifications to be applied to a beam angle (or a set of beam angles) for display to a user and/or as an input to various other downstream applications. The beam angles may already be defined in a base treatment plan but modified based on the output of the machine learning model.

In an embodiment, a computer-implemented method may comprise executing, by at least one processor, a machine learning model that receives an input of data associated with a treatment plan for a patient and outputs a beam angle for the patient indicating a direction of radiation into the patient, wherein the machine learning model is trained using a training dataset comprising a training treatment plan and a corresponding score, wherein the machine learning model iteratively calculates a reward, using a policy, for a possible beam angle for the training treatment plan in the training dataset; and transmitting, by the at least one processor, the beam angle to a second processor.

The treatment plan and the training treatment plan may comprise at least one of a medical image, a clinical goal, a planning target volume, an organ at risk, a radiation type, a radiation dose, an initial beam angle, or a field geometry.

The medical image may include at least a structure of the planning target volume or a structure of the organ at risk.

The computer-implemented method may further comprise executing the machine learning model that receives the input of data associated with the treatment plan for the patient and outputs a field geometry, wherein the machine learning model is trained using a training dataset comprising the training treatment plan and a corresponding score.

The computer-implemented method may further comprise presenting, by the processor, for display, the beam angle.

The machine learning model may be trained using asynchronous advantage actor critic reinforcement learning.

The machine learning model may be implemented using hybrid graphics processing units and central processing units.

The machine learning model may be optimized with respect to one or more clinical goals received in the treatment plan, the clinical goals including at least one of a dosimetric quality, a robustness measure, metrics based on linear energy transfer, or relative biological effects.

The computer-implemented method may further comprise receiving, from the second processor, a revised treatment plan, wherein the revised treatment plan is based on the beam angle; executing, by the at least one processor, the machine learning model using the revised treatment plan for the patient and outputting a revised beam angle; and transmitting, by the at least one processor, the revised beam angle to the second processor.

Iteratively calculating the reward, using the policy, for the possible beam angle from the training treatment plan in the training dataset may include iteratively comparing the reward to a baseline.

In another embodiment, a system may comprise a server comprising a processor and a non-transitory computer-readable medium containing instructions that when executed by the processor causes the processor to perform operations comprising: execute a machine learning model that receives an input of data associated with a treatment plan for a patient and outputs a beam angle for the patient indicating a direction of radiation into the patient, wherein the machine learning model is trained using a training dataset comprising a training treatment plan and a corresponding score, wherein the machine learning model iteratively calculates a reward, using a policy, for a possible beam angle for the training treatment plan in the training dataset; wherein the machine learning model iteratively increases a summation of rewards until the policy satisfies an accuracy threshold; transmit the beam angle to a second processor.

At least one of the treatment plan or the training treatment plan may comprise at least one of a medical image, a clinical goal, a planning target volume, an organ at risk, a radiation type, a radiation dose, an initial beam angle, or a field geometry.

The medical image may include at least a structure of the planning target volume or a structure of the organ at risk.

The processor may be further configured to execute the machine learning model that receives the input of data associated with the treatment plan for the patient and outputs a dose distribution, wherein the machine learning model is trained using a training dataset comprising the training treatment plan and a corresponding score.

The processor may be further configured to present for display, the beam angle.

The machine learning model may be trained using asynchronous advantage actor critic reinforcement learning.

The machine learning model may be implemented using hybrid graphics processing units and central processing units.

The machine learning model may be optimized with respect to one or more clinical goals received in the treatment plan, the clinical goals including at least one of a dosimetric quality, a robustness measure, metrics based on linear energy transfer, or relative biological effects.

The processor may be further configured to receive, from the second processor, a revised treatment plan, wherein the revised treatment plan is based on the beam angle; execute the machine learning model using the revised treatment plan for the patient and outputting a revised beam angle; and transmit the revised beam angle to the second processor.

Iteratively calculating the reward, using the policy, for the possible beam angle from the training treatment plan in the training dataset may include iteratively comparing the reward to a baseline.

## BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.

FIG. 1 illustrates components of a beam angle optimization system, according to an embodiment.
FIG. 2 illustrates a flow diagram of a process executed in a beam angle optimization system, according to an embodiment.
FIG. 3A illustrates an example of a simplified reinforcement learning model, according to an embodiment.
FIG. 3B illustrates an example of an asynchronous advantage actor critic reinforcement learning model, according to an embodiment.
FIG. 4 illustrates an example recommendation of beam angles based on a treatment plan, according to an embodiment.
FIG. 5 illustrates a non-limiting visual example of a workflow utilizing the methods and systems described herein, according to an embodiment.
FIG. 6 illustrates another non-limiting visual example of a workflow utilizing the methods and systems described herein, according to an embodiment.

## DETAILED DESCRIPTION

Reference will now be made to the illustrative embodiments depicted in the drawings, and specific language will be used here to describe the same. The invention is defined in the following claims.

Radiotherapy clinics may utilize software solutions for executing radiation therapy. The software solutions may analyze patient data, clinical goals, and a multitude of other factors to generate a customized radiation treatment plan (e.g.,

dose distribution, radiation parameters, side effect prediction, organ and/or tumor segmentation, machine therapy attributes, treatment frequency, treatment timing, treatment modalities). The radiation parameters may include an optimized beam angle for a patient and one or more numbers of beams (e.g., field geometry settings). The radiation parameters may be improved (selected, recommended, and/or optimized) based on considering one or more clinical goals such as a dosimetric goodness function, a robustness, biological effects of radiation, among others.

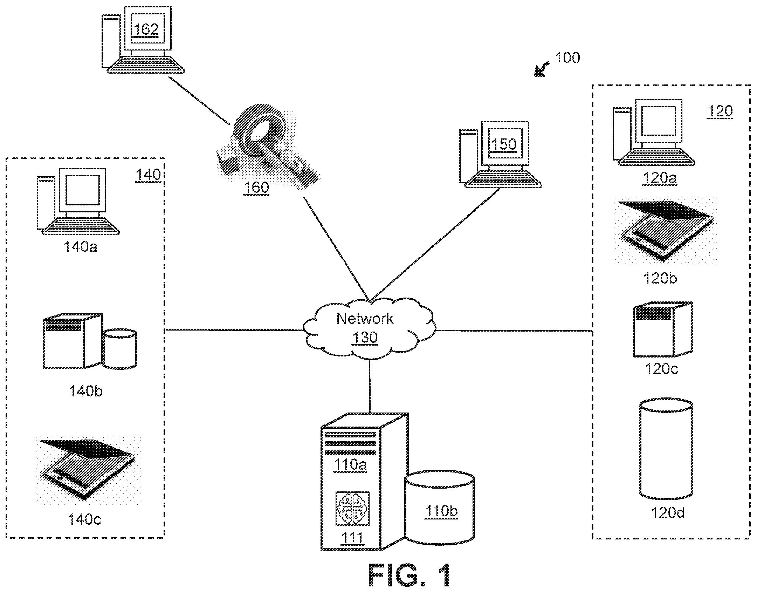

**FIG. 1** illustrates components of beam angle optimization system **100,** according to an embodiment. The system **100** may include an analytics server **110a,** system database **110b,** machine learning models **111,** electronic data sources **120a-d** (collectively electronic data sources **120),** end-user devices **140a-c** (collectively end-user devices **140),** an administrator computing device **150,** and a medical device **160** having a medical device computer **162.** Various components depicted in **FIG. 1** may belong to a radiotherapy clinic at which patients may receive radiotherapy treatment, in some cases via one or more radiotherapy machines located within the clinic (e.g., medical device **160).** The above-mentioned components may be connected to each other through a network **130.** Examples of the network **130** may include, but are not limited to, private or public LAN, WLAN, MAN, WAN, and the Internet. The network **130** may include wired and/or wireless communications according to one or more standards and/or via one or more transport mediums.

The communication over the network **130** may be performed in accordance with various communication protocols such as Transmission Control Protocol and Internet Protocol (TCP/IP), User Datagram Protocol (UDP), and IEEE communication protocols. In one example, the network **130** may include wireless communications according to Bluetooth specification sets or another standard or proprietary wireless communication protocol. In another example, the network **130** may also include communications over a cellular network, including, e.g., a GSM (Global System for Mobile Communications), CDMA (Code Division Multiple Access), EDGE (Enhanced Data for Global Evolution) network.

The system **100** is not confined to the components described herein and may include additional or other components, not shown for brevity, which are to be considered within the scope of the embodiments described herein.

The analytics server **110a** may generate and display an electronic platform configured to use various computer models **111** (including artificial intelligence and/or machine learning models) to identify radiation parameters. More specifically, the platform may display one or more optimized (recommended, identified, and/or selected) beam angles and/or a number of recommended beam angles (and/or the associated optimized beam angle). The number of recommended beam angles may be considered the field geometry settings for external beam radiotherapy. The electronic platform may include graphical user interface (GUI) displayed on each electronic data source **120,** the end-user devices **140,** the administrator computing device **150,** and/or the medical device computer **162.** An example of the electronic platform generated and hosted by the analytics server **110a** may be a web-based application or a website configured to be displayed on different electronic devices, such as mobile devices, tablets, personal computers, and the like.

In a non-limiting example, a medical professional may input patient attributes, medical images, PTV and/or OAR information, radiotherapy treatment parameters (e.g., initial beam angles, field geometries, dose distributions, and the like) to electronic data source **120b.** The medical professional operating devices **120b, 140c,** and **162,** may access the platform, review displayed revised beam angles (or sets of beam angles or other radiation therapy treatment parameters) generated from the machine learning model **111.** Additionally or alternatively, the operations invoked by the analytics server **110a** to optimize beam angle may be part of the operations in a sequence of operations to optimize a patient treatment plan (e.g., dose distribution among the patient's organs). The medical professional may use the medical professional device (e.g., medical professional device **140c)** as both a device to display results predicted by the analytics server **110a** and in some cases used as an electronic data source (e.g., electronic data source **120b)** to train the machine learning models **111.**

The analytics server **110a** may recommend beam angles and other radiation parameters and/or treatment plan attributes used for proton radiation, photon radiation, and electron radiation. In particular, analytics server **110a** may utilize the methods and systems described herein to automatically learn and recommend an improved (or optimized) beam angle. The analytics server **110a** may display the beam angle and other radiation parameters and/or treatment plan attributes on an end-user device **140c,** medical computing device **162,** and/or a medical professional device **120b.** The analytics server **110a** may also use the beam angles and other radiation parameters and/or treatment plan attributes via one or more downstream applications. Further, the analytics server **110a** may transmit the beam angles and other radiation parameters and/or treatment plan attributes to one or more other servers (e.g., clinic server **140b).** Additionally, or alternatively, the analytics server **110a** (or other server) may adjust the configuration of one of end-user devices **140** (e.g., the end-user device **140c)** based on the optimized beam angle.

The analytics server **110a** may host a website accessible to users operating any of the electronic devices described herein (e.g., end users, medical professionals), where the content presented via the various webpages may be controlled based upon each particular user's role or viewing permissions. The analytics server **110a** may be any computing device comprising a processor and non-transitory machine-readable storage capable of executing the various tasks and processes described herein. The analytics server **110a** may employ various processors such as central processing units (CPU) and graphics processing unit (GPU), among others. Non-limiting examples of such computing devices may include workstation computers, laptop computers, server computers, and the like. While the system **100** includes a single analytics server **110a,** the analytics server **110a** may include any number of computing devices operating in a distributed computing environment, such as a cloud environment.

The analytics server **110a** may execute software applications configured to display the electronic platform (e.g., host a website), which may generate and serve various webpages to each electronic data source **120** and/or end-user devices **140.** Different users may use the website to view and/or interact with the recommended (optimized) results. Different servers, such as server **120c** and clinic server **140b** may also use the recommended results in downstream processing. Additionally, or alternatively, the analytics server **110a** may use the recommended beam angle to optimize one or more other radiation parameters and/or treatment plan attributes. For example, the analytics server **110a** may use the recommended beam angle to optimize a dose distribution.

The analytics server **110a** may be configured to require user authentication based upon a set of user authorization credentials (e.g., username, password, biometrics, cryptographic certificate, and the like). The analytics server **110a** may access the system database **110b** configured to store user credentials, which the analytics server **110a** may be configured to reference in order to determine whether a set of entered credentials (purportedly authenticating the user) match an appropriate set of credentials that identify and authenticate the user.

The analytics server **110a** may generate and host webpages based upon a particular user's role within the system **100.** In such implementations, the user's role may be defined by data fields and input fields in user records stored in the system database **110b** of the analytics server **110a.** The analytics server **110a** may authenticate the user and may identify the user's role by executing an access directory protocol (e.g., LDAP). The analytics server **110a** may generate webpage content that is customized according to the user's role defined by the user record in the system database **110b.**

The analytics server **110a** may receive medical images from a user or retrieve such data from a data repository, analyze the data, and display the results on the electronic platform. For instance, in a non-limiting example, the analytics server **110a** may query and retrieve medical images from the database **120d** and combine the medical images with segment data received from a medical professional operating the medical professional device **120b.** Additionally, or alternatively, the analytics server **110a** may segment the medical image automatically or perform other pre-processing steps on the medical image captured from the medical device **160.**

The analytics server **110a** may also perform other pre-processing steps on the medical image captured from the medical device **160.** The analytics server **110a** may execute various machine learning models **111** (stored within the system database of the clinic server **140b** or the analytics server **110b)** to analyze the retrieved data. The analytics server **110a** may then display the results via the electronic platform on the administrator computing device **150,** the medical professional device **120b,** medical computing device **162** and/or the end-user devices **140.**

The electronic data sources **120** may represent various electronic data sources that contain, retrieve, and/or input data associated with a patient's treatment plan including patient data and treatment data. For instance, the analytics server **110a** may use the clinic computer **120a,** medical professional device **120b,** server **120c** (associated with a physician and/or clinic), and/or database **120d** (associated with the physician and/or the clinic) to retrieve/receive data associated with the patient's treatment plan.

End-user devices **140** may be any computing device comprising a processor and a non-transitory machine-readable storage medium capable of performing the various tasks and processes described herein. Non-limiting examples of an end-user device **140** may be a workstation computer, laptop computer, tablet computer, and server computer. In operation, various users may use end-user devices **140** to access the GUI operationally managed by the analytics server **110a.** Specifically, the end-user devices **140** may include clinic computer **140a,** clinic server **140b,** and a medical device professional **140c.** Even though referred to herein as "end user" devices, these devices may not always be operated by end users. For instance, the clinic server **140b** may not be directly used by an end user. However, the results stored onto the clinic server **140b** may be used to populate various GUIs accessed by an end user via the medical professional device **140c.**

The administrator computing device **150** may represent a computing device operated by a system administrator. The administrator computing device **150** may be configured to display beam angles, radiation parameters and/or other radiation therapy treatment attributes generated by the analytics server **110a** (e.g., various analytic metrics determined during training of one or more machine learning models and/or systems); monitor various models **111** utilized by the analytics server **110a,** electronic data sources **120,** and/or end-user devices **140;** review feedback; and/or facilitate training or retraining (calibration) of the machine learning models **111** that are maintained by the analytics server **110a.**

The medical device **160** may be a radiotherapy machine (e.g., a linear accelerator, particle accelerator (including circular accelerators), or a cobalt machine)) configured to implement a patient's radiotherapy treatment. The medical device **160** may also include an imaging device capable of emitting radiation such that the medical device **160** may perform imaging according to various methods to accurately image the internal structure of a patient. For instance, the medical device **160** may include a rotating system (e.g., a static or rotating multi-view system). A non-limiting example of a multi-view system may include a stereo systems (e.g., two systems may be arranged orthogonally). The medical device **160** may also be in communication with a medical device computer **162** that is configured to display various GUIs discussed herein. For instance, the analytics server **110a** may display the results predicted by the machine learning model **111** onto the medical device computer **162.**

In operation, a physician or other medical professional may access an application executing on the medical professional device **120b** and input patient data and the patient's treatment data (e.g., patient information, patient diagnosis, radiation therapy radiation requirements and/or thresholds). The analytics server **110a** then uses a patient identifier to query patient data (e.g., patient anatomy and/or medical images) from the electronic data sources **120.** The analytics server may then identify a clinic associated with the patient (e.g., clinic performing the treatment) and retrieve one or more files associated with treatment templates and clinic rules. The analytics server **110a** may then utilize the systems and methods described herein to generate optimized radiation parameters (e.g., beam angles or a treatment plan, in some configurations) and forward the optimized beam angles, other radiation parameters and/or treatment plan attributes to one or more downstream applications. The downstream application may, for example, employ additional machine learning models **111** to optimize a treatment plan based on the optimized beam angle. Additionally, or alternatively, the analytics server **110a** may present for display the optimized beam angle results onto the medical professional device **120b,** clinic computer **140a,** medical computing device **162,** and/or the medical device **160** (e.g., a display screen of the radiotherapy machine).

The analytics server **110a** may be in communication (real-time or near real-time) with the computing device **162,** end-user device **140** and/or electronic data sources **120,** such that a server/computer hosting the medical device **160** can adjust the medical device **160** based on the beam angles, treatment attributes and/or radiation parameters revised by the analytics server **110a.** For instance, the radiotherapy machine may adjust the gantry, beam blocking device (e.g. multi leaf collimator MLC), and couch based on optimized beam angles, where the optimized beam angle is an angle of the medical device **160** that emits radiation in a direction that maximizes radiation to PTVs and minimizes radiation to OARs. The analytics server **110a** may transmit instructions to the radiotherapy machines indicating any number or type of radiation parameters and/or treatment attributes to facilitate such adjustments.

The analytics server **110a** may store machine learning models **111** (e.g., neural networks, random forest, support vector machines, or other deep learning models), that are trained to predict (and improve or optimize) beam angles that optimize the treatment plan with respect to various clinical goals of patients at radiotherapy clinics. The analytics server **110a** may train the machine learning models **111** using patient data and treatment data associated with patients who were previously treated. For instance, the analytics server **110a** may receive patient data (e.g., physical attributes, diagnoses, 3D medical images) and treatment data recommended from one or more medical professionals (beam angles, does distributions, a number of beams for treatment, radiation type, radiation intensity) from any of the data sources **120** and clinical goals.

Machine learning models **111** may be stored in the system database **110b** and may correspond to individual radiotherapy clinics or otherwise different sets of radiotherapy machines (e.g., radiotherapy machines that are located at individual radiotherapy clinics, are located in different geographical regions, treat specific types of diseases (e.g., different types of cancer), treat specific genders, etc.). For example, the machine learning model **111** may be associated with an identifier indicating the radiotherapy clinic, set of radiotherapy machines, or a specific disease for which it is configured to predict the probability of a reference point of a template image being at a location in 3D space.

A medical professional at a radiotherapy clinic may access an end-user device **140** located at the clinic or access an account associated with the clinic. The medical professional may provide an input at a user interface that causes the end-user device **140** to transmit a request to access a machine learning model **111** that is associated with the clinic and/or the radiotherapy machines located within the clinic. The request may include an identifier associated with the machine learning model **111,** the clinic, a treatment plan generated by the one or more medical professionals, and/or the set of radiotherapy machines that the analytics server **110a** may use as a key in a look-up table to identify the machine learning model **111.** The analytics server **110a** may receive the request and, in some cases, after authenticating the user, identify the machine learning model **111** via the identifier. The analytics server **110a** may transmit the identified machine learning model **111** to the end-user device **140** or send an alert indicating the end-user device is authorized to access the model(s) **111.** Upon receipt or access to the machine learning model **111,** the end user device **140** may perform the systems and methods described herein to train or retrain the machine learning model **111** to predict (and improve or optimize) beam angles.

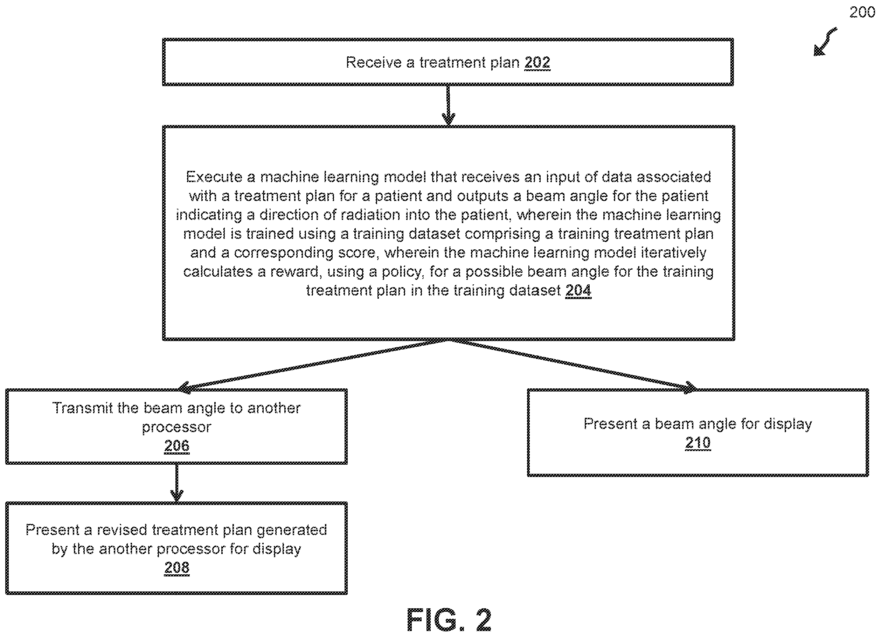

FIG. 2 illustrates a flow diagram of a process executed in a beam angle optimization system, according to an embodiment. The method **200** includes steps for determining a revised (optimized, improved, modified, identified, selected, and/or predicted) beam angle, set of beam angles or other radiation parameters and/or treatment attributes, according to an embodiment. As described herein, inputs and outputs may be described in the singular (e.g., ingest a medical image or output a revised beam angle). It should be appreciated that multiple inputs (e.g., medical images and corresponding PTV/OAR structures in the medical images) and multiple outputs (e.g., a set of beam angles, a set of radiation parameters, and the like) are considered. For example, treatment plans may consist of multiple treatment beans, where each treatment beam has a different angle. Accordingly, the method **200** may simultaneously improve all of the beam angles (and/or other radiation parameters) associated with a particular patient and a particular treatment plan. The method **200** may include steps **202-210.** However, other embodiments may include additional or alternative steps, or may omit one or more steps altogether.

The method **200** is described as being executed by an analytics server, such as the analytics server described in **FIG. 1.** The analytics server may employ one or more CPUs and GPUs to perform one or more steps of method **200.** The CPUs and/or GPUs may be performed in part by the analytics server and in part by one or more other servers and/or computing devices. The servers and/or computing devices employing the CPUs and GPUs may be local and/or remote (or some combination). For example, one or more virtual machines in a cloud may employ one or more CPUs and GPUs to perform one or more steps of method **200.** A hybrid CPU and GPU implementation may improve the speed associated with training a machine learning model to select a beam angle. However, one or more steps of method **200** may be executed by any number of computing devices operating in the distributed computing system described in **FIG. 1.** For instance, one or more computing devices may locally perform part or all of the steps described in **FIG. 2.** Moreover, an "agent," referring to the learner or the trainer (e.g., the analytics server training the machine learning model or the machine learning model itself), may perform one or more steps discussed herein.

In step **202,** the analytics server may receive a treatment plan from one or more sources (e.g., user devices such as physician device **120b,** end-user devices **140** including the radiotherapy machine **140d,** databases **110b,** and electronic data sources **120** in **FIG. 1).** For instance, the analytics server may query one or more databases to identify medical data associated with the patient. The analytics server may query data associated with the patient's anatomy, such as physical data (e.g., height, weight, and/or body mass index) and/or other health-related data (e.g., blood pressure or other data relevant to the patient receiving radiation therapy treatment). The analytics server may also retrieve data associated with current and/or previous medical treatments received by the patient (e.g., data associated with the patient's previous surgeries).

The analytics server may analyze the data received and may generate additional queries accordingly. For instance, the analytics server may retrieve data associated with one or more medical (or other) devices needed for the patient. The analytics server may retrieve data indicating that the patient suffers from a respiratory medical condition. As a result, the analytics server may generate and transmit a query to one or more electronic data sources to identify whether the patient uses/needs a ventilator.

If necessary, the analytics server may also analyze the patient's medical data records to identify the needed patient attributes. For instance, the analytics server may query a database to identify the patient's body mass index (BMI). However, because many medical records are not digitalized, the analytics server may not receive the patient's BMI value using simple query techniques. As a result, the analytics server may retrieve the patient's electronic health data and may execute one or more analytical protocols (e.g., natural language processing) to identify the patient's body mass index. In another example, if the analytics server does not receive PTV and/or OAR data, then the analytics server may execute various image recognition protocols and segmentation to identify the PTV and/or OAR data in various medical images (e.g., planning images, simulation images, and/or diagnostic images). For example, the analytics server may automatically segment and/or pre-process various medical images using trained machine learning models. The machine learning models trained to segment medical images may generate contours on the medical images, segmenting the medical image and identifying one or more PTVs and/or OARs.

The treatment plan may be the plan determined by one or more medical professionals to treat a particular patient using radiation therapy. The treatment plan may include information such as a dose distribution, radiation parameters such as beam angles, field geometry settings, side effect prediction, organ and/or tumor segmentation, machine therapy attributes such as gantry position, couch position, beam blocking devices, treatment frequency, treatment timing, and/or treatment modalities, among others. The treatment plan may include a set of structures (e.g., PTVs and/or OARs) segmented from medical images and at least one defined beam angle.

The treatment plan may also include one or more medical images. The analytics server may transform or otherwise convert the medical image into a point cloud representation of structures (e.g., PTVs and/or OARs) in the medical images. An automatic image segmentation module may automatically segment PTVs and/or OARs in medical images. The medical images may be segmented, or otherwise analyzed to identify different contrasts in the medical image.

In some configurations, the medical images may be two-dimensional (2D). In other configurations, the medical images may be three-dimensional (3D). If the medical images are 2D, the analytics server may convert the 2D images into 3D images (e.g., using triangulation protocols, photogrammetry). Medical images may include CT scans, 4D CT scans, MRIs, and X-ray images, among others.

The treatment plan may also include one or more clinical goals. Clinical goals may include a dosimetric goodness function (e.g., a dosimentric quality or metric), a robustness measure, relative biological effects, and linear energy transfer metrics, among others. A medical professional may indicate one or more particular clinical goals for particular patients. For example, a medical professional may identify that a clinical goal associated with a younger patient is a robust treatment plan because the young patient may be more likely to move during treatment than an older patient. Additionally, or alternatively, clinical goals may include dose thresholds for PTVs and/or OARs. Clinical goals may also be associated with a particular clinic and/or particular radiotherapy machines. Additionally, or alternatively, a clinical goal may be predetermined (e.g., determined via an administrator computing device **150 in FIG. 1).**

At step **204,** the analytics server executes a machine learning model that receives an input of data associated with a treatment plan for a patient and outputs a set of beam angles for the patient indicating a set of directions of radiation into the patient. If the treatment plan contains an initial set of beam angles for the patient, the analytics server may output an improved (or optimized, recommended, selected, identified, revised) set of beam angles for the patient (e.g., a tensor indicating revised modifications to be applied to a beam angle).

By exploiting the fact that the core policy network of the machine learning model implicitly forecasts other radiation parameters and/or treatment plan attributes, machine learning models may be repurposed (e.g., retrained) to directly predict (or improve, select, recommend, and/or optimize) radiation parameters and/or treatment plan attributes such as dose distribution and/or dose intensity (e.g., dose prediction). Additionally, or alternatively, the analytics server may output one or more improved (or revised, optimized, selected, identified) other radiation treatment parameters such as a field geometry.

In some configurations, the treatment plan may not include an initial beam angle or other initial radiation parameter and/or treatment attribute. Accordingly, the analytics server will output a beam angle (e.g., not a revised beam angle because there is no beam angle to revise). That is, the analytics server may output a beam angle independent of receiving an initial beam angle in the treatment plan.

In other configurations, the analytics server may revise other treatment plan attributes (e.g., dose distribution) using other outputted treatment plan information (e.g., the revised beam angle). The analytics server may also output other treatment plan attributes. That is, the analytics server may output a treatment attribute independent of receiving various initial treatment attributes in the treatment plan.

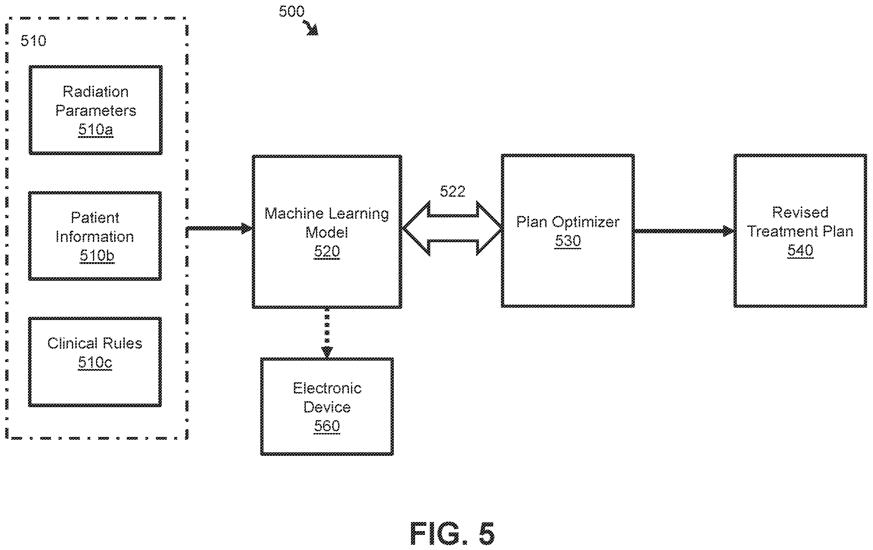

The executed machine learning model may be trained to revise (or optimize) beam angles for a patient using a training dataset including data ingested from previously performed treatments including treatment plans with corresponding medical images, radiation parameters (beam angles, field geometries), clinical goals, and a corresponding score representing a clinical quality, where the score is generated (e.g., by the analytics server and/or a second processor, such as plan optimizer **530** in **FIG. 5)** and the like. The analytics server and/or second processor may determine the score based on the clinical goals, dose distributions, beam angles, field geometries, of previously performed treatments. Additionally, or alternatively, a medical professional may score the clinical outcome of previously performed treatments.

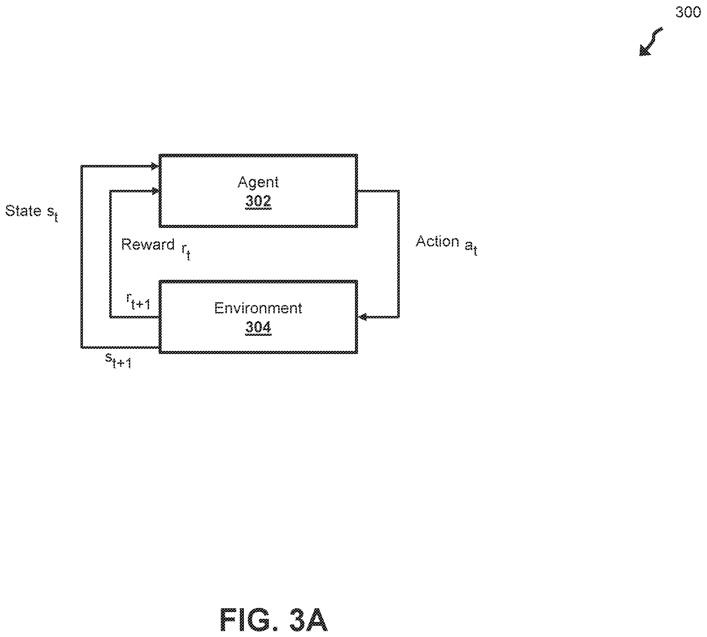

**FIG. 3A** illustrates a simplified reinforcement learning model **300,** according to an embodiment. As used herein, the "agent" refers to the learner or the trainer (e.g., the analytics server training the machine learning model or the machine learning model itself). Agents use an initial treatment plan setup, attempt modifications (revisions, improvements, and/or optimizations) on parts of the treatment plan (e.g., radiation parameters, and in particular beam angles), learn whether the modifications were beneficial, and propose a set of modifications (e.g., recommended, improved, optimized beam angles) based on the trained reinforcement learning model **300.**

In reinforcement learning, an agent **302** interacts with an environment **304.** As discussed herein, an agent **302** refers to the learner or trainer. The environment **304** refers to encapsulated anatomical structures of the patient, as well as the positioning of the radiation fields in the initial treatment plan. At each time step $t$ (e.g., each iteration), the agent **302** observes a state $s_t$ and selects an action from a set of actions. The possible set of actions may include increasing or decreasing a beam angle, not modifying a beam angle, and increasing or decreasing multiple beam angles simultaneously. The beam angles in the possible set of actions (e.g., action space) that may be associated with a particular position of the radiotherapy machine (e.g., a linear accelerator implemented in the medical device).

The possible set of actions (e.g., action space) may also be arbitrarily defined and depend on the solution space considerations. For example, the solution space may be discretized such that the possible angles for beams are at fixed intervals rather than on a continuous range. Accordingly, the action space may include actions such as "move in one direction," "move in another direction," or "don't modify y beam" where y is a specific beam. Additionally, or alternatively, the action space may include actions such as "move x steps in one direction," "move x steps in the other direction," or "don't modify y beam."

The action space may include more complex schemes such as dual step-sizes for an explore/exploit approach. For example, the action space may include actions such as "move a small step in one direction," "move a big step in one direction," "move a small step in the other direction," "move a big step in the other direction," and "don't modify y beam."

In other examples, the solution space may be continuous rather than discrete. For example, the action space may include actions such as "move x degrees" or "do not modify the beam position of y beam." In the event a continuous solution space is implemented, the agents **302** may need to train for longer such that the agents **302** can determine, for example, in which direction there might be a better plan and how far in a certain direction a better plan may be.

Agents **302** may select an action based on the value of taking each action, where the value of selecting the action is defined as the expected reward received when taking that action from the possible set of actions. Agents **302** may select actions based on exploratory actions and exploitation actions. An exploratory action improves an agent's knowledge about an action by using the explored action in a sequence resulting in a reward calculation. An exploitation action is a "greedy" action that exploits the agent's **302** current action-value estimates. Using epsilon-greedy action selection, for example, the agent **302** balances exploratory actions and exploitation actions. The agent **302** may select an epsilon value and perform an exploitation action or an exploratory action based on the value of the epsilon and one or more exploitation and/or exploration thresholds. The agent **302** may randomly select an epsilon value and/or select an epsilon value from a predetermined distribution of epsilon values.

Agents **302** may also select an action using a policy $\pi$, where $\pi$ maps states (and observations) to actions. The policy $\pi$ gives the probability of taking a certain action when the agent is in a certain state.

In response to selecting an action (or multiple actions), the environment **304** may change, and there may be a new state $s_{t+1}$. The agent **302** may receive feedback, indicating how the action affected the environment **304**. In some configurations, the agent **302** determines the feedback. In other configurations, the analytics server may provide feedback. In yet other configurations, a second processor (e.g., plan optimizer **530 in FIG. 5)** may provide feedback.

The agent **302** learns (e.g., reconfigures its policy $\pi$) by taking actions and analyzing the rewards received. A reward functions can include, for example, $R(s_t)$, $R(s_t, a_t)$, and $R(s_t, a_t, s_{t+1})$. In some configurations, the reward may be a dosimetric goodness function. For example, a reward function based on a dosimetric goodness function may include various quadratic terms representing considerations determined by a dosimetrist. Reward functions may also be based on other clinical goals including a robustness measure, relative biological effects, and/or linear energy transfer metrics, among others. Additionally or alternatively, a second processor (e.g., plan optimizer **530** in **FIG. 5)** may calculate rewards based on clinical goals, dosimetric goodness functions, robustness measures, relative biological effects, and/or linear energy transfer metrics, among others, and provide the feedback to the agent **302.**

Each iteration (or after multiple iterations and/or steps), the agent **302** selects a policy $\pi$ (and an action) based on the current state $s_t$ and the agent **302** (or the machine learning model) calculates a reward. Each iteration, the agent **302** (or machine learning model) iteratively increases a summation of rewards.

One goal of reinforcement learning is to determine a policy $\pi$ that maximizes the cumulative set of rewards, determined via the reward function. A core policy network evaluates the environment **304** and produces probabilistic distributions that the agent **302** (or the analytics server) uses to select how to modify the beam fields for a given possible beam angle.

The analytics server weighs policies based on the rewards determined at each step (or series of steps) such that certain policies (and actions) are encouraged and/or discouraged in response to the environment **304** being in a certain state. The policies are optimized by taking the gradient of an objective function (e.g., a reward function) to maximize a cumulative sum of rewards at each step, or after a predetermined number of steps (e.g., a delayed reward).

In some configurations, the rewards at each step may be compared (e.g., on an iterative basis) to a baseline. The baseline may be an expected performance (e.g., beam angle or other radiation parameter of the received treatment plan), or an average performance (e.g., an average beam angle over a series of steps). Evaluating a difference between the baseline and the reward is considered evaluating a value of advantage (or advantage value). The value of the advantage indicates how much better the reward is from the baseline (e.g., instead of an indication of which actions were rewarded and which actions were penalized).

In an example of training using reinforcement learning, a second processor (e.g., a plan optimizer **530** in **FIG. 5)** may determine a score using clinical goals (or other metrics). For example, the second processor may determine a score based on an initial treatment plan (e.g., a template treatment plan, a treatment plan proposed by a medical professional) by evaluating the initial treatment plan according to how well the clinical goals are met. The score assigned by the second processor may be used as the baseline by the agents **302** or the machine learning model. The machine learning model may compare a score associated with the treatment plan using the revised beam angle to the baseline score to evaluate whether the action selected by the agents **302** according to the policy should be punished or rewarded. Additionally or alternatively, the analytics server (including the agents **302** in the machine learning model) may determine and use a score based on the clinical goals.

Additionally or alternatively, the analytics server (including the agents **302** in the machine learning model or a different processor and/or different server) may measure the rewards (and/or associated beam angles) against metrics to assess the quality of the revised beam angles (sets of revised beam angles, or other radiation parameters and/or treatment information) and update the central policy network. For example, the baseline may be an expected reward. The analytics server (or a different process such as the plan optimizer **530** in **FIG. 5)** may evaluate the baseline by evaluating, for instance, a base treatment plan. The evaluation of the base treatment plan may include assigning a clinical quality score to the base treatment plan. The analytics server (or different processor such as the plan optimizer **530** in **FIG. 5** and/or agents **302)** may compare the evaluated base treatment plan to a revised treatment plan using the revised beam angles. Accordingly, the baseline may be expected clinical goals that can be correlated with the clinical quality of the revised treatment plan (or revised treatment parameter).

Additionally or alternatively, the analytics server (including the agents **302** in the machine learning model or a different processor and/or different server) may prioritize other metrics such as a measure of plan robustness, linear energy transfer (LET), and/or relative biological effects (RBE) rather than dosimetric quality and physical dose (or some combination).

The agents **302** trains themselves by choosing the action(s) based on policies that provide the highest cumulative set of rewards. The agents **302** of the machine learning model may continue training until a predetermined threshold has been satisfied. For instance, the analytics server may train the machine learning model until the advantage value is within a predetermined accuracy threshold. Additionally or alternatively, the analytics server may continue training the machine learning model until a predetermined number of steps (or series of steps called episodes, or iterations) have been reached.

The analytics server may determine weights to maximize the objective function (e.g., reward function) during training as shown in Equation 1 below.

$$\nabla_\theta J(\theta) = \frac{1}{m} \sum_{i=1}^{m} \sum_{t=0}^{T} \nabla_\theta log \pi_\theta(a_t|s_t)((Q(s_t, a_t) - V_\theta(s_t)) \qquad \text{Equation (1)}$$

$log\pi_\theta (a_t |s_t)$ is an agent term
$Q(s_t, a_t) - V_\theta(s_t)$ is a critic term
T is a number of steps in an episode
m is a number of episodes

In Equation 1 above, agents **302** may approximate both the value function $V_\theta(s_t)$ in the critic term and the policy function $log\pi_\theta(a_t|s_t)$ of the agent term. The agent term represents the probability of selecting a policy of taking an action $a_t$ given the state $s_t$. The state-action value Q function in the critic term gives the expectation of the cumulative reward according to the current state $s_t$ after taking an action $a_t$ according to the current policy $\pi$. The value function in the critic term gives the expectation of the cumulative rewards at each step. The critic term may be approximated using Q actor-critic techniques, advantage actor-critic techniques, temporal difference (TD) actor-critic techniques, TD actor-critic techniques with a discount value, and/or natural actor-critic techniques, among others.

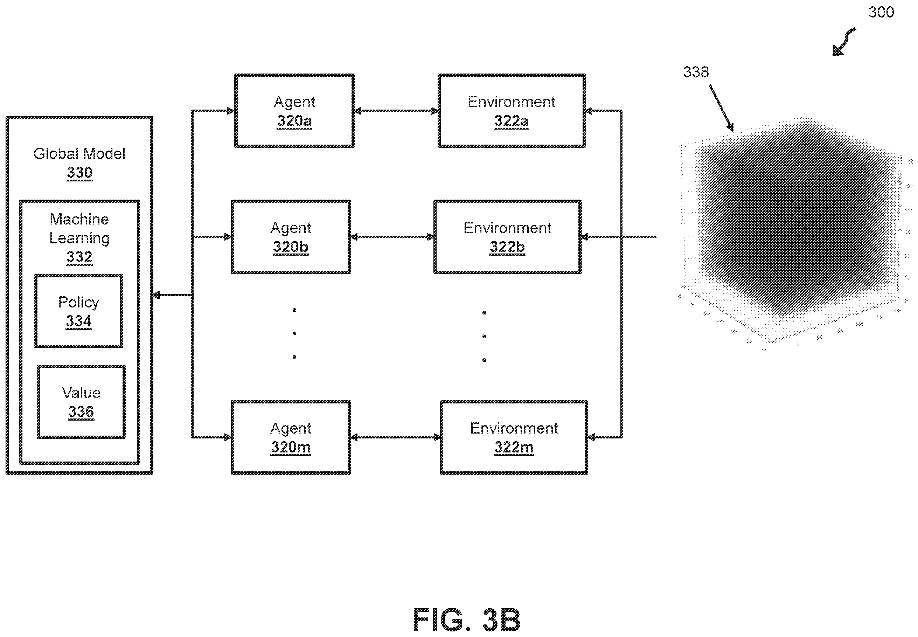

**FIG. 3B** illustrates asynchronous advantage actor critic reinforcement learning model **300,** according to an embodiment. In model **300,** the analytics server utilizes various asynchronous agents **320a, 320b,** and **320m** (collectively called agents **320)** with each agent **320** having a corresponding environment **322a, 322b,** and **322m** (collectively called environment **322).** The analytics server may employ a GPU to instantiate multiple learning agents in parallel. Environment **322** may be based on 3D point cloud **338,** where the 3D point cloud **338** is based on a medical image. The 3D point cloud **338** may be a tensor-base point cloud representation of the structures (PTVs, OARs) in the patient's body. The environment **322** may be an encapsulation of the data used for each agent **320.** The analytics server may convert information in the treatment plan into a serializable representation. For example, the environment **322** may include a copy of the 3D point cloud **338** and beam angle vector data (e.g., received from a treatment plan).

Each agent asynchronously performs actions and calculates rewards using a single machine learning model **332** (such as a deep neural network) in a global model **330.** The analytics server may configure the machine learning model **332** to include sufficient layers to capture identifiers associated with various points in the 3D point cloud **338.** That is, the layers of the machine learning model **332** may be used to determine relationships of the points in the 3D point cloud **338** and various PTV and/or OAR radiation sensitivities.

In some configurations, policies **334** and action values **336** are updated every step (or predetermined number of steps) based on the cumulative rewards determined by each agent **320.** Action values **336** may be the values used in tandem with the policy **334** to act as a critic. Each agent may contribute to the global policy **334** and value **336** such that the total knowledge of the global model **330** increases and the global policy **334** learns how to best modify the treatment plans to achieve higher treatment performances. Each time the global model **330** is updated (e.g., after every step and/or predetermined number of steps), the analytics server propagates new weights back to agents **320** such that each agent shares common policies **334** and values **336.**

The global model **330** allows each agent to have a more diversified training data and eliminates a need for synchronization of models associated with each agent **320.** In other configurations, there may be models associated with each agent **320** and each agent may calculate a reward using a corresponding machine learning model.

In some configurations, the analytics server may update the global model **330** using agents **320** operating in other servers. That is, the analytics server may employ agents on other servers (e.g., via federated learning) to update the global model **330** and corresponding policy **334** and value **336.**

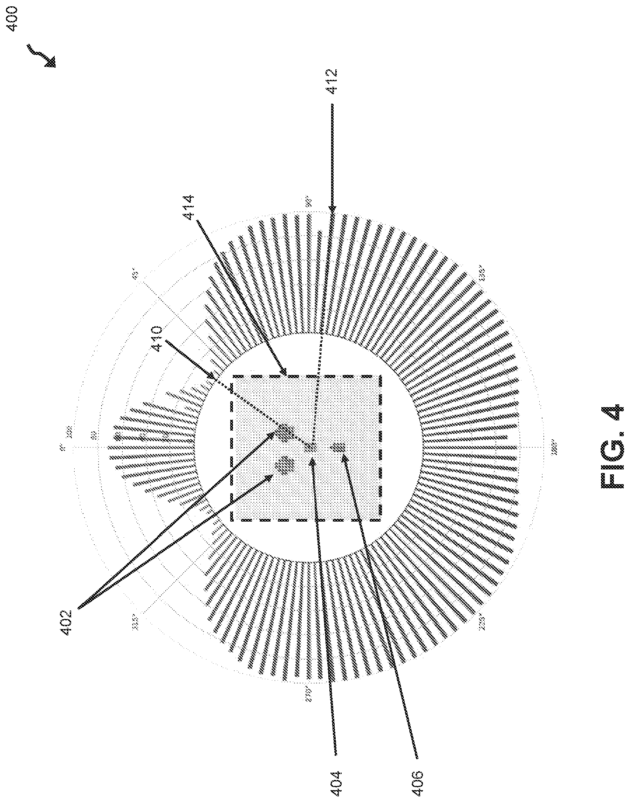

**FIG. 4** illustrates a recommendation **400** of beam angles based on a treatment plan, according to an embodiment. For simplicity, a 2D image of a patient is used to generate a 2D point cloud **414.** In other configurations, the image used to generate the 2D point cloud **414** may be synthetically defined from a geometric case. The 2D point cloud **414** includes points associated with the patient's eyes (e.g., points **402),** points associated with the tumor (e.g., points **404),** and points associated with the patient's spine (e.g., points **406).** In response to receiving treatment plan information (e.g., including a medical image, clinical goals, PTV information, OAR information, and/or radiation dose information), the analytics server may determine one or more beam angles. Recommendation **400** illustrates a full pan of beam angles and corresponding rewards. A beam angle with a low reward is a beam angle that did not perform well with respect to one or more clinical goals. For example, a baseline beam angle may be associated with a higher reward than the beam angle determined by the analytics server.

In some configurations, the dosimetric goodness function describes tissue sensitivity, bone sensitivity, tumor sensitivity, and the like using, for example, a weighted sum of quadratic terms. The analytics server would have learned, during training, to avoid revising beam angles to go through points in the point cloud **414** associated with certain identifying information (e.g., OAR sensitivity information). Accordingly, beam angles indicating a direction of radiation into the patient through the patient's eyes (indicated by points **402**) would not receive a high reward because points **402** may be associated with OAR sensitivity information. As shown, the analytics server determined that a 36 degree beam angle (indicated at **410**) received a low reward, likely because the beam angle includes a direction of radiation through the patient's eyes (indicated by points **402**) to irradiate the tumor (indicated by points **410**). In contrast, the analytics server may determine that a 96 degree beam angle (indicated at **412**) receives a high reward, likely because the beam angle does not include a direction of radiation through the patient's eyes (indicated by points **402**) or spine (indicated by points **406**).

Referring back to **FIG. 2,** in step **210,** the analytics server may present for display a predetermined number of beam angles (e.g., a top five beam angles). In some configurations, the analytics server may present for display the full pan of beam angle evaluations (e.g., recommendation **400** in **FIG. 4**).

In step **206,** the analytics server may transmit the beam angle (or set of beam angles) to another processor (or another computer). Referring to **FIG. 5,** a non-limiting visual example of a workflow utilizing the methods and systems described herein is illustrated. In this example **500,** the analytics server provides beam angles to a plan optimizer **530** (e.g., a second processor) to provide a suggested (improved) treatment plan that is optimized for a patient. As discussed herein, the suggested treatment plan may be a revised (improved) version of the received treatment plan (e.g., step **202** in **FIG. 2**) based on beam angles determined by the machine learning model **520** (e.g., a first processor).

The analytics server may first receive a treatment plan for a particular patient **510.** The treatment plan may include beam angles or other radiation parameters **510a** (e.g., field geometries, dosage information), patient information **510b** (e.g., medical images, PTVs, OARs), and/or clinical goals **510c** (e.g., dosimetric goodness function, robustness metrics, biological effects of radiation, metrics based on linear energy transfer). The analytics server may train a machine-learning model **520** using previously performed radiation therapy treatments and corresponding scores representing a clinical quality. The trained machine-learning model **520** may then identify various beam angles to optimize (or improve, revise, select, predict, or identify) associated with the treatment plan for the particular patient **510.**

The machine-learning model **520** may transmit the optimized (or improved, revised, selected, predicted or identified) beam angle for display to an electronic device **560.** The electronic device may include a radiotherapy machine, a patient device, or an administrator device, among others.

The optimized beam angle determined via the machine learning model **520** may also be ingested by the plan optimizer **530.** The plan optimizer **530** may be a treatment planning and/or monitoring software solution. The plan optimizer **530** may be executed by a second processor. For example, the plan optimizer **530** may be executed on a second computer. Additionally, or alternatively, the plan optimizer **530** may be executed on the processor executing machine learning model **520.** The machine learning models **520** may be an ad-hoc external software capable of working in tandem with the plan optimizer **530.**

The plan optimizer **530** may analyze various factors associated with the patient and the patient's treatment to generate and/or optimize (revise) a treatment plan for the patient (e.g., field geometry, treatment modality, dosage distribution, dosage prescription, and/or radiation parameters). One of the factors considered by the plan optimizer **530** may be the beam angle outputted (identified, predicted, selected) by the machine-learning model **520.**

While the plan optimizer **530** may consider beam angles as a factor, the plan optimizer **530** may weigh the beam angles differently than other factors considered to optimize (or generate) the patient's revised treatment plan. For instance, the revised treatment plan **540** determined by the plan optimizer **530** may not be dictated by the beam angles predicted by the machine-learning model **520.** The plan optimizer **530** may utilize various cost function analysis protocols where the beam angles is evaluated in light of the other (sometimes more important) factors. In some cases, other factors may be prioritized over the beam angles.

The plan optimizer **530** may iteratively revise the patient's treatment plan by iteratively revising different attributes of the patient's treatment plan (e.g., field geometry, dose distribution). With each iteration, the plan optimizer **530** may transmit revised treatment plan data back to the machine-learning model **530** whereby the machine-learning model **530** can recalculate/re-predict new beam angles based on the revised treatment data generated by the plan optimizer (iteration **522).** The plan optimizer **530** and the machine-learning model **520** may repeat the iteration **522** until the patient's treatment plan is optimized. When the plan optimizer **530** completes the patient's treatment plan, the plan optimizer **330** may transmit the revised (optimized, suggested) treatment plan **540** to one or more electronic devices where a user (e.g., medical professional) can review the revised treatment plan. For instance, the analytics server may display the revised treatment plan **540** on a computer of a clinic where a radiotherapy technician or a treating oncologist can review the treatment plan.

Generating a revised treatment plan may include evaluating a cost function. The objective of radiation therapy is to apply dosage satisfying a treatment threshold to a patient's PTV without applying dosage satisfying a harmful threshold to the patient's OAR. The plan optimizer **530** balances the amount of dosage received by OAR against the importance of the dosage to be applied to the PTV. In some cases, the plan optimizer **530** determines an acceptable amount of residual dosage at an OAR, such that PTV receives a proper amount of dosage. The plan optimizer **530** may minimize the dosage received by a patient's OAR (also referred to herein as the cost) and weigh the amount of dosage received against the dosage received by the patient's PTV.

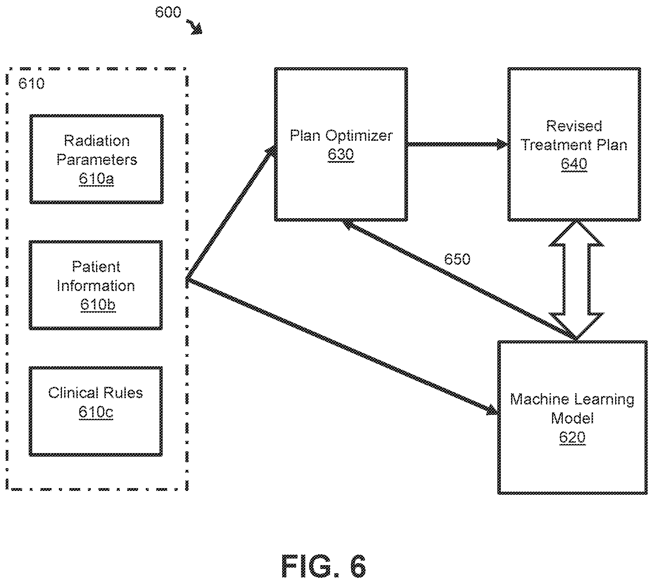

Referring now to **FIG. 6,** another non-limiting visual example of a workflow utilizing the methods and systems described herein is illustrated. In this example **600,** the analytics server uses the methods discussed herein to evaluate a plan suggested (and improved) by a plan optimizer **630.** In the depicted embodiment, the plan optimizer **630** and a machine-learning model **620** may work independently (as opposed to working together, as depicted in **FIG. 5).** The plan optimizer **630** may be executed by a second processor. For example, the plan optimizer **630** may be executed on a second computer. Additionally, or alternatively, the plan optimizer **630** may be executed on the processor executing the machine learning model **620.**

The analytics server may first receive a treatment plan for a particular patient **610.** As described above, the treatment plan **610** may include radiation parameters **610a** (e.g., beam angles), patient information **610b** (e.g., medical images), and clinical goals **610c** (e.g., dosimetric goodness function, robustness metrics, biological effects of radiation, metrics based on linear energy transfer). The analytics server may train the machine-learning model **620** using previously performed radiation therapy treatments and a corresponding score representing a clinical quality, based on previously selected beam angles. The analytics server may then transmit the revised beam angles to the plan optimizer **630** where the plan optimizer **630** uses various analytical protocols and cost functions to generate a score to evaluate the revised beam angles. The plan optimizer may also optimize (or generate) a revised treatment plan for the patient **640** using the treatment plan **610.**

The analytics server may then transmit the score, treatment plan **610** and/or the revised treatment plan **640** to the trained machine-learning model **620.** The trained machine-learning model **620** may then use the score and the methods described herein to calculate revised (optimized, improved) beam angles for a treatment plan (e.g., treatment plan **610** and/or revised treatment plan **640).**

Additionally or alternatively, the trained machine-learning model **620** (or the analytics server) may transmit one or more revised radiation parameters values including beam angles back to the plan optimizer **630** (step **650).** The plan optimizer **630** may then use the revised radiation parameters to recalculate a treatment plan for the patient, generate a score, and/or optimize a revised treatment plan **640** accordingly. Upon the plan optimizer **630** optimizing a revised treatment plan **640,** the trained machine-learning model **620** may re-evaluate the revised treatment plan **640** using the methods described herein and a score produced by the plan optimizer **630,** re-revising beam angles (or other radiation parameters). The plan optimizer **630** and the trained machine-learning model **620** may iteratively repeat this process where with each iteration the plan optimizer **630** updates the revised treatment plan **640** and the trained machine-learning model **620** re-evaluates the revised treatment plan **640** using the score. This iterative process may continue until the trained machine-learning model **620** determines that the revised treatment plan **640** is within tolerable thresholds (e.g., clinical goals, scores).

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of this disclosure or the claims.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions may be stored as one or more instructions or code on a non-transitory computer-readable or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the principles defined herein may be applied to other embodiments without departing from the spirit or scope of the subject matter disclosed herein. Thus, the present disclosure is not intended to be limited to the embodiments shown herein but is to be accorded the widest scope consistent with the following claims and the principles and novel features disclosed herein.

While various aspects and embodiments have been disclosed, the invention is defined the following claims.

## Claims

1. A computer-implemented method of beam angle optimization comprising:

   executing, by at least one processor, a machine learning model that receives an input of data associated with a treatment plan for a patient and outputs a beam angle for the patient indicating a direction of radiation into the patient,

   wherein the machine learning model is trained using a training dataset comprising a training treatment plan and a corresponding score,
   wherein the machine learning model iteratively calculates a reward, using a policy, for a possible beam angle for the training treatment plan in the training dataset, and
   wherein the machine learning model iteratively increases a summation of rewards until the policy satisfies an accuracy threshold; and

   transmitting, by the at least one processor, the beam angle to a second processor.

2. The computer-implemented method according to claim 1, wherein at least one of the treatment plan or the training treatment plan comprise at least one of a medical image, a clinical goal, a planning target volume, an organ at risk, a radiation type, a radiation dose, an initial beam angle, or a field geometry;
   and optionally:
   wherein the medical image includes at least a structure of the planning target volume or a structure of the organ at risk.

3. The computer-implemented method according to any preceding claim, further comprising executing the machine learning model that receives the input of data associated with the treatment plan for the patient and outputs a dose distribution, wherein the machine learning model is trained using a training dataset comprising the training treatment plan and the corresponding score.

4. The computer-implemented method according to any preceding claim, further comprising presenting, by the processor, for display, the beam angle.

5. The computer-implemented method according to any preceding claim, wherein the machine learning model is trained using asynchronous advantage actor critic reinforcement learning;
   and/or:
   wherein the machine learning model is implemented using hybrid graphics processing units and central processing units.

6. The computer-implemented method according to any preceding claim, wherein the machine learning model is optimized with respect to one or more clinical goals received in the treatment plan, the clinical goals including at least one of a dosimetric quality, a robustness measure, metrics based on linear energy transfer, or relative biological effects.

7. The computer-implemented method according to any preceding claim, further comprising:

   receiving, by the at least one processor from the second processor, a revised treatment plan, wherein the revised treatment plan is based on the beam angle;
   executing, by the at least one processor, the machine learning model using the revised treatment plan for the patient and outputting a revised beam angle; and
   transmitting, by the at least one processor, the revised beam angle to the second processor.

8. The computer-implemented method according to any preceding claim, wherein iteratively calculating the reward, using the policy, for the possible beam angle from the training treatment plan in the training dataset includes iteratively comparing the reward to a baseline.

9. A system comprising:
   a server comprising a processor and a non-transitory computer-readable medium containing instructions that when executed by the processor causes the processor to perform operations comprising:

   execute a machine learning model that receives an input of data associated with a treatment plan for a patient and outputs a beam angle for the patient indicating a direction of radiation into the patient, wherein the machine learning model is trained using a training dataset comprising a training treatment plan and a corresponding score, wherein the machine learning model iteratively calculates a reward, using a policy, for a possible beam angle for the training treatment plan in the training dataset, wherein the machine learning model iteratively increases a summation of rewards until the policy satisfies an accuracy threshold; and
   transmit the beam angle to a second processor.

10. The system according to claim 9, wherein at least one of the treatment plan or the training treatment plan comprise at least one of a medical image, a clinical goal, a planning target volume, an organ at risk, a radiation type, a radiation dose, an initial beam angle, or a field geometry;
    and optionally:
    wherein the medical image includes at least a structure of the planning target volume or a structure of the organ at risk.

11. The system according to any of claim 9 or claim 10, wherein the processor is further configured to execute the machine learning model that receives the input of data associated with the treatment plan for the patient and outputs a dose distribution, wherein the machine learning model is trained using a training dataset comprising the training treatment plan and a corresponding score.

12. The system according to any of claim 9 to claim 11, wherein the processor is further configured to present for display, the beam angle.

13. The system according to any of claim 9 to claim 12, wherein the machine learning model is trained using asynchronous advantage actor critic reinforcement learning;
    and/or:
    wherein the machine learning model is implemented using hybrid graphics processing units and central processing units.

14. The system according to any of claim 9 to claim 13, wherein the machine learning model is optimized with respect to one or more clinical goals received in the treatment plan, the clinical goals including at least one of a dosimetric quality, a robustness measure, metrics based on linear energy transfer, or relative biological effects.

15. The system according to any of claim 9 to claim 14, wherein the processor is further configured to:

    receive, from the second processor, a revised treatment plan, wherein the revised treatment plan is based on the beam angle;
    execute the machine learning model using the revised treatment plan for the patient and outputting a revised beam angle; and
transmit the revised beam angle to the second processor;
and/or:
wherein iteratively calculating the reward, using the policy, for the possible beam angle from the training treatment plan in the training dataset includes iteratively comparing the reward to a baseline.

**Patentansprüche**

1. Computerimplementiertes Verfahren zur Strahlwinkeloptimierung, umfassend:

   Ausführen, durch mindestens einen Prozessor, eines Maschinenlernmodells, das eine Eingabe von Daten empfängt, die mit einem Behandlungsplan für einen Patienten assoziiert sind, und einen Strahlwinkel für den Patienten ausgibt, der eine Strahlungsrichtung in den Patienten angibt,
   wobei das Maschinenlernmodell unter Verwendung eines Trainingsdatensatzes trainiert wird, der einen Trainingsbehandlungsplan und eine entsprechende Bewertung umfasst,
   wobei das Maschinenlernmodell iterativ eine Belohnung für einen möglichen Strahlwinkel für den Trainingsbehandlungsplan in dem Trainingsdatensatz unter Verwendung einer Richtlinie berechnet und
   wobei das Maschinenlernmodell iterativ eine Summe von Belohnungen erhöht, bis die Richtlinie einen Genauigkeitsschwellenwert erfüllt; und
   Übertragen, durch den mindestens einen Prozessor, des Strahlwinkels an einen zweiten Prozessor.

2. Computerimplementiertes Verfahren nach Anspruch 1, wobei mindestens einer von dem Behandlungsplan oder dem Trainingsbehandlungsplan mindestens eines von einem medizinischen Bild, einem klinischen Ziel, einem Planungszielvolumen, einem gefährdeten Organ, einem Bestrahlungstyp, einer Strahlendosis, einem anfänglichen Strahlwinkel oder einer Feldgeometrie umfasst;
   und optional:
   wobei das medizinische Bild mindestens eine Struktur des Planungszielvolumens oder eine Struktur des gefährdeten Organs beinhaltet.

3. Computerimplementiertes Verfahren nach einem der vorhergehenden Ansprüche, ferner umfassend Ausführen des Maschinenlernmodells, das die Eingabe von Daten empfängt, die mit dem Behandlungsplan für den Patienten assoziiert sind, und eine Dosisverteilung ausgibt, wobei das Maschinenlernmodell unter Verwendung eines Trainingsdatensatzes trainiert wird, der den Trainingsbehandlungsplan und die entsprechende Bewertung umfasst.

4. Computerimplementiertes Verfahren nach einem der vorhergehenden Ansprüche, ferner umfassend Darstellen, durch den Prozessor zur Anzeige, des Strahlwinkels.

5. Computerimplementiertes Verfahren nach einem der vorhergehenden Ansprüche, wobei das Maschinenlernmodell unter Verwendung von asynchronem Vorteilsfaktor-kritischem Verstärkungslernen trainiert wird;
   und/oder:
   wobei das Maschinenlernmodell unter Verwendung von hybriden Grafikverarbeitungseinheiten und zentralen Verarbeitungseinheiten implementiert wird.

6. Computerimplementiertes Verfahren nach einem der vorhergehenden Ansprüche, wobei das Maschinenlernmodell in Bezug auf ein oder mehrere klinische Ziele, die in dem Behandlungsplan aufgenommen werden, optimiert wird, wobei die klinischen Ziele mindestens eines von einer dosimetrischen Qualität, einem Robustheitsmaß, Metriken, die auf linearer Energieübertragung basieren, oder relativen biologischen Effekten beinhalten.

7. Computerimplementiertes Verfahren nach einem der vorhergehenden Ansprüche, ferner umfassend:

   Empfangen, durch den mindestens einen Prozessor von dem zweiten Prozessor, eines überarbeiteten Behandlungsplans, wobei der überarbeitete Behandlungsplan auf dem Strahlwinkel basiert;
   Ausführen, durch den mindestens einen Prozessor, des Maschinenlernmodells unter Verwendung des überarbeiteten Behandlungsplans für den Patienten und Ausgeben eines überarbeiteten Strahlwinkels; und
   Übertragen, durch den mindestens einen Prozessor, des überarbeiteten Stahlwinkels an den zweiten Prozessor.

8. Computerimplementiertes Verfahren nach einem der vorhergehenden Ansprüche, wobei das iterative Berechnen der Belohnung, unter Verwendung der Richtlinie, für den möglichen Strahlwinkel anhand des Trainingsbehandlungsplans in dem Trainingsdatensatz iteratives Vergleichen der Belohnung mit einer Basislinie umfasst.

9. System, umfassend:

einen Server, der einen Prozessor und ein nichttransitorisches computerlesbares Medium umfasst, das Anweisungen enthält, die, wenn sie durch den Prozessor ausgeführt werden, den Prozessor dazu veranlassen, Vorgänge durchzuführen, die Folgendes umfassen:

Ausführen eines Maschinenlernmodells, das eine Eingabe von Daten empfängt, die mit einem Behandlungsplan für einen Patienten assoziiert sind, und einen Strahlwinkel für den Patienten ausgibt, der eine Strahlungsrichtung in den Patienten angibt, wobei das Maschinenlernmodell unter Verwendung eines Trainingsdatensatzes trainiert wird, der einen Trainingsbehandlungsplan und eine entsprechende Bewertung umfasst, wobei das Maschinenlernmodell iterativ eine Belohnung, unter Verwendung einer Richtlinie, für einen möglichen Strahlwinkel für den Trainingsbehandlungsplan in dem Trainingsdatensatz berechnet, wobei das Maschinenlernmodell iterativ eine Summe von Belohnungen erhöht, bis die Richtlinie einen Genauigkeitsschwellenwert erfüllt; und
Übertragen des Strahlwinkels an einen zweiten Prozessor.

10. System nach Anspruch 9, wobei mindestens einer von dem Behandlungsplan oder dem Trainingsbehandlungsplan mindestens eines von einem medizinischen Bild, einem klinischen Ziel, einem Planungszielvolumen, einem gefährdeten Organ, einem Bestrahlungstyp, einer Strahlendosis, einem anfänglichen Strahlwinkel oder einer Feldgeometrie umfasst;
und optional:
wobei das medizinische Bild mindestens eine Struktur des Planungszielvolumens oder eine Struktur des gefährdeten Organs beinhaltet.

11. System nach einem von Anspruch 9 oder Anspruch 10, wobei der Prozessor ferner dazu konfiguriert ist, das Maschinenlernmodell auszuführen, das die Eingabe von Daten empfängt, die mit dem Behandlungsplan für den Patienten assoziiert sind, und eine Dosisverteilung ausgibt, wobei das Maschinenlernmodell unter Verwendung eines Trainingsdatensatzes trainiert wird, der den Trainingsbehandlungsplan und eine entsprechende Bewertung umfasst.

12. System nach einem von Anspruch 9 bis Anspruch 11, wobei der Prozessor ferner dazu konfiguriert ist, den Strahlwinkel zur Anzeige bereitzustellen.

13. System nach einem von Anspruch 9 bis Anspruch 12, wobei das Maschinenlernmodell unter Verwendung von asynchronem Vorteilsfaktor-kritischem Verstärkungslernen trainiert wird;
und/oder:
wobei das Maschinenlernmodell unter Verwendung von hybriden Grafikverarbeitungseinheiten und zentralen Verarbeitungseinheiten implementiert wird.

14. System nach einem von Anspruch 9 bis Anspruch 13, wobei das Maschinenlernmodell in Bezug auf ein oder mehrere klinische Ziele, die in dem Behandlungsplan aufgenommen werden, optimiert wird, wobei die klinischen Ziele mindestens eines von einer dosimetrischen Qualität, einem Robustheitsmaß, Metriken, die auf linearer Energieübertragung basieren, oder relativen biologischen Effekten beinhalten.

15. System nach einem von Anspruch 9 bis Anspruch 14, wobei der Prozessor ferner zu Folgendem konfiguriert ist:

Empfangen, von dem zweiten Prozessor, eines überarbeiteten Behandlungsplans, wobei der überarbeitete Behandlungsplan auf dem Strahlwinkel basiert;
Ausführen des Maschinenlernmodells unter Verwendung des überarbeiteten Behandlungsplans für den Patienten und Ausgeben eines überarbeiteten Strahlwinkels; und
Übertragen des überarbeiteten Strahlwinkels an den zweiten Prozessor;
und/oder:
wobei das iterative Berechnen der Belohnung, unter Verwendung der Richtlinie, für den möglichen Strahlwinkel anhand des Trainingsbehandlungsplans in dem Trainingsdatensatz iteratives Vergleichen der Belohnung mit einer Basislinie beinhaltet.

**Revendications**

1. Procédé mis en œuvre par ordinateur d'optimisation d'angle de faisceau, comprenant :

   l'exécution, par au moins un processeur, d'un modèle d'apprentissage automatique qui reçoit une entrée de données associée à un plan de traitement pour un patient et délivre un angle de faisceau pour le patient indiquant une direction de rayonnement dans le patient,
   dans lequel le modèle d'apprentissage automatique est entraîné à l'aide d'un ensemble de données d'entraînement comprenant un plan de traitement d'entraînement et un score correspondant,
   dans lequel le modèle d'apprentissage automatique calcule de manière itérative une récompense, à l'aide d'une politique, pour un angle de faisceau possible pour le plan de traitement d'entraînement dans l'ensemble de données d'entraînement, et
   dans lequel le modèle d'apprentissage automatique augmente de manière itérative une somme de récompenses jusqu'à ce que la politique satisfasse un seuil de précision ; et
   la transmission, par l'au moins un processeur, de l'angle de faisceau à un second processeur.

2. Procédé mis en œuvre par ordinateur selon la revendication 1, dans lequel au moins l'un du plan de traitement ou du plan de traitement d'entraînement comprend au moins l'un d'une image médicale, d'un objectif clinique, d'un volume cible de planification, d'un organe à risque, d'un type de rayonnement, d'une dose de rayonnement, d'un angle de faisceau initial ou d'une géométrie de champ ;
   et éventuellement :
   dans lequel l'image médicale comporte au moins une structure du volume cible de planification ou une structure de l'organe à risque.

3. Procédé mis en œuvre par ordinateur selon une quelconque revendication précédente, comprenant également l'exécution du modèle d'apprentissage automatique qui reçoit l'entrée de données associée au plan de traitement pour le patient et délivre une distribution de dose, dans lequel le modèle d'apprentissage automatique est entraîné à l'aide d'un ensemble de données d'entraînement comprenant le plan de traitement d'entraînement et le score correspondant.

4. Procédé mis en œuvre par ordinateur selon une quelconque revendication précédente, comprenant également la présentation, par le processeur, pour affichage, de l'angle de faisceau.

5. Procédé mis en œuvre par ordinateur selon une quelconque revendication précédente, dans lequel le modèle d'apprentissage automatique est entraîné à l'aide d'un apprentissage par renforcement critique d'acteur d'avantage asynchrone ;
   et/ou :
   dans lequel le modèle d'apprentissage automatique est mis en œuvre à l'aide d'unités de traitement graphique hybrides et d'unités centrales de traitement.

6. Procédé mis en œuvre par ordinateur selon une quelconque revendication précédente, dans lequel le modèle d'apprentissage automatique est optimisé par rapport à un ou plusieurs objectifs cliniques reçus dans le plan de traitement, les objectifs cliniques comportant au moins l'un d'une qualité dosimétrique, d'une mesure de robustesse, de métriques basées sur un transfert d'énergie linéaire ou d'effets biologiques relatifs.

7. Procédé mis en œuvre par ordinateur selon une quelconque revendication précédente, comprenant également :

   la réception, par l'au moins un processeur à partir du second processeur, d'un plan de traitement révisé, dans lequel le plan de traitement révisé est basé sur l'angle de faisceau ;
   l'exécution, par l'au moins un processeur, du modèle d'apprentissage automatique à l'aide du plan de traitement révisé pour le patient et en délivrant un angle de faisceau révisé ; et
   la transmission, par l'au moins un processeur, de l'angle de faisceau révisé au second processeur.

8. Procédé mis en œuvre par ordinateur selon une quelconque revendication précédente, dans lequel le calcul itératif de la récompense, à l'aide de la politique, pour l'angle de faisceau possible à partir du plan de traitement d'entraînement dans l'ensemble de données d'entraînement comporte la comparaison itérative de la récompense à une ligne de base.

**9.** Système comprenant :
un serveur comprenant un processeur et un support non transitoire lisible par ordinateur contenant des instructions qui, lorsqu'elles sont exécutées par le processeur, amènent le processeur à réaliser des opérations comprenant :

exécuter un modèle d'apprentissage automatique qui reçoit une entrée de données associée à un plan de traitement pour un patient et délivre un angle de faisceau pour le patient indiquant une direction de rayonnement dans le patient, dans lequel le modèle d'apprentissage automatique est entraîné à l'aide d'un ensemble de données d'entraînement comprenant un plan de traitement d'entraînement et un score correspondant, dans lequel le modèle d'apprentissage automatique calcule de manière itérative une récompense, à l'aide d'une politique, pour un angle de faisceau possible pour le plan de traitement d'entraînement dans l'ensemble de données d'entraînement, dans lequel le modèle d'apprentissage automatique augmente de manière itérative une somme de récompenses jusqu'à ce que la politique satisfasse un seuil de précision ; et
transmettre l'angle de faisceau à un second processeur.

**10.** Système selon la revendication 9, dans lequel au moins l'un du plan de traitement ou du plan de traitement d'entraînement comprend au moins l'un d'une image médicale, d'un objectif clinique, d'un volume cible de planification, d'un organe à risque, d'un type de rayonnement, d'une dose de rayonnement, d'un angle de faisceau initial ou d'une géométrie de champ ;
et éventuellement :
dans lequel l'image médicale comporte au moins une structure du volume cible de planification ou une structure de l'organe à risque.

**11.** Système selon l'une quelconque de la revendication 9 ou de la revendication 10, dans lequel le processeur est également configuré pour exécuter le modèle d'apprentissage automatique qui reçoit l'entrée de données associée au plan de traitement pour le patient et délivre une distribution de dose, dans lequel le modèle d'apprentissage automatique est entraîné à l'aide d'un ensemble de données d'entraînement comprenant le plan de traitement d'entraînement et un score correspondant.

**12.** Système selon l'une quelconque de la revendication 9 à la revendication 11, dans lequel le processeur est également configuré pour présenter pour affichage l'angle de faisceau.

**13.** Système selon l'une quelconque de la revendication 9 à la revendication 12, dans lequel le modèle d'apprentissage automatique est entraîné à l'aide d'un apprentissage par renforcement critique d'acteur d'avantage asynchrone ;
et/ou :
dans lequel le modèle d'apprentissage automatique est mis en œuvre à l'aide d'unités de traitement graphique hybrides et d'unités centrales de traitement.

**14.** Système selon l'une quelconque de la revendication 9 à la revendication 13, dans lequel le modèle d'apprentissage automatique est optimisé par rapport à un ou plusieurs objectifs cliniques reçus dans le plan de traitement, les objectifs cliniques comportant au moins l'un d'une qualité dosimétrique, d'une mesure de robustesse, de métriques basées sur un transfert d'énergie linéaire ou d'effets biologiques relatifs.

**15.** Système selon l'une quelconque de la revendication 9 à la revendication 14, dans lequel le processeur est également configuré pour :

recevoir, à partir du second processeur, un plan de traitement révisé, dans lequel le plan de traitement révisé est basé sur l'angle de faisceau ;
exécuter le modèle d'apprentissage automatique à l'aide du plan de traitement révisé pour le patient et en délivrant un angle de faisceau révisé ; et
transmettre l'angle de faisceau révisé au second processeur ;
et/ou :
dans lequel le calcul itératif de la récompense, à l'aide de la politique, pour l'angle de faisceau possible à partir du plan de traitement d'entraînement dans l'ensemble de données d'entraînement comporte la comparaison itérative de la récompense à une ligne de base.

FIG. 1

200

Receive a treatment plan 202

Execute a machine learning model that receives an input of data associated with a treatment plan for a patient and outputs a beam angle for the patient indicating a direction of radiation into the patient, wherein the machine learning model is trained using a training dataset comprising a training treatment plan and a corresponding score, wherein the machine learning model iteratively calculates a reward, using a policy, for a possible beam angle for the training treatment plan in the training dataset 204

Transmit the beam angle to another processor
206

Present a beam angle for display
210

Present a revised treatment plan generated by the another processor for display
208

FIG. 2

State $s_t$

Reward $r_t$

$r_{t+1}$

$s_{t+1}$

Agent
302

Action $a_t$

Environment
304

300

FIG. 3A

EP 4 359 070 B1

**FIG. 3B**

**FIG. 4**

EP 4 359 070 B1

FIG. 5

FIG. 6

**REFERENCES CITED IN THE DESCRIPTION**

**Non-patent literature cited in the description**

- Deep BOO! Automating Beam Orientation Optimization in Intensity-Modulated Radiation Therapy. **OGUNMOLU, O.** ; **FOLKERTS, M.** ; **NGUYEN, D.** ; **GANS, N.** ; **JIANG, S.** Algorithmic Foundations of Robotics XIII. WAFR 2018. Springer Proceedings in Advanced Robotic. Springer, 2020, vol. 14